Europäisches Patentamt

European Patent Office

Office européen des brevets

(11) Veröffentlichungsnummer: **0 607 886 A1**

## (12) EUROPÄISCHE PATENTANMELDUNG

(21) Anmeldenummer: **94100522.5**

(22) Anmeldetag: **14.01.94**

(51) Int. Cl.5: **A61K 7/46**, A61K 7/48

(30) Priorität: **19.01.93 DE 4301266**

(43) Veröffentlichungstag der Anmeldung:
**27.07.94 Patentblatt 94/30**

(84) Benannte Vertragsstaaten:
**BE CH DE FR GB IT LI NL**

(71) Anmelder: **Metzger, Wolfgang**
**Am Schönblick 24**
**D-99425 Weimar(DE)**

(72) Erfinder: **Metzger, Wolfgang**
**Am Schönblick 24**
**D-99425 Weimar(DE)**

(74) Vertreter: **Patentanwälte Grünecker,**
**Kinkeldey, Stockmair & Partner**
**Maximilianstrasse 58**
**D-80538 München (DE)**

(54) Neue Parfümkomplex-Zusammensetzung mit verbesserter Substantivität sowie deren Verwendung.

(57) Die Erfindung betrifft eine neue Parfümkomplex-Zusammensetzung, die mindestens ein Parfümöl, mindestens ein Silikatmaterial zur reversiblen Adsorption des Parfümöls, mindestens ein Alkylammoniumsalz und mindestens eine ungesättigte Fettsäure enthält. Die Parfümkomplex-Zusammensetzung zeichnet sich durch ein maximales Parfümaufziehvermögen, eine ausgezeichnete Haftbarkeit und eine zeitlich dosierte Abgabe über einen langen Zeitraum aus und kann wirkungsvoll in Weichspülern, Feinwaschmitteln mit Weicheffekt, Wäschestärken, Duftspülern, Shampoos, Cremeschaumbädern, medizinischen Duschbädern, Zahnpasten, Handreinigungsmitteln, Papierpräparationsmitteln und abrassiven Reinigern verwendet werden.

EP 0 607 886 A1

Rank Xerox (UK) Business Services
(3.10/3.09/3.3.4)

Die vorliegende Erfindung betrifft eine neue, substantive Parfümkomplex-Zusammensetzung und deren Verwendung.

Parfümöle natürlichen oder synthetischen Ursprungs finden als Duftstoffe umfangreiche Anwendung in technischen, haushaltstechnischen und kosmetischen Formulierungen. Sie dienen dazu, den Eigengeruch der Zusammensetzung zu maskieren bzw. darüber hinaus den Warenverkauf durch den suggestiv wirkenden illusionsfördernden Duft zu stimulieren.

Duftnoten sollten vor allem in Waschmitteln, Wäschenachbehandlungsmitteln, kosmetischen Formulierungen, aber auch in technischen und haushaltstechnischen Produkten möglichst lange auf den behandelten Gegenständen bzw. Medien haften, d.h., sie sollten substantiv wirken.

Herkömmliche Parfümkompositionen bestehen daher aus den folgenden drei Teilen: 1. einer Kopfnote (leichtflüchtige Bestandteile meist frischen Charakters); 2. einer Mittelnote (mäßig flüchtige Bestandteile) und 3. einer Basisnote (wenig flüchtige Riechstoffe, die den Grundcharakter bestimmen).

Die Basisnote enthält normalerweise Fixateure, die für eine gut haftende Duftbeständigkeit verantwortlich sind. Die folgenden vier Gruppen, die die Verdunstungszeit der Parfümöle verlangsamen, sind bekannt:

1. Tierische Rohstoffe, wie Ambra, Castoreum, Moschus, Zibet oder Maskon;

2. Eigenfixateure, die von "Natur" aus eine schwere Note besitzen, z.B. Rosenöl und Lavendelöl;

3. Pseudofixateure (verschiedene spezielle organische Verbindungen, vor allem Diethylenglykolmethylether), die geringste Wirksamkeit besitzen und vor allem der Werbung dienen.

4. "Echte" Fixateure, wie Styrax, Labdanum, Benzoe, Iris und Eichenmoos, die durch Adsorptionskräfte und Wasserstoffbrücken wirken.

Der Aufbau v.a. dieser "echten" Fixateure ist sehr kompliziert und der Einsatz derartigen organischen Materials erscheint aus ökonomischen und ökologischen Gründen bedenklich.

Bisher konnte mit den bekannten Parfümölen, besonders dann, wenn Duft funktioneller Selbstzweck ist, keine ausreichende Haftung erzielt werden, und die Abgabe der Parfümöle, vor allem von der Haut, war auch nicht zufriedenstellend dosiert. Schließlich lassen sich bekannte Parfümöle, bzw. sie enthaltende Produkte, auch nicht leicht genug auf u.a. Haut, Haar und Wäsche aufziehen.

Es ist demnach Aufgabe der vorliegenden Erfindung, eine Parfümkomplex-Zusammensetzung zur Verfügung zu stellen, mit der der Einsatz der wertvollen und teuren tierischen und pflanzlichen organischen Rohstoffe durch Erhöhung des Parfümaufziehvermögens und der temporären Haftbarkeit von Parfümölen (Substantivität) verringert, der Einsatz von weniger gut haftenden Parfümölen bzw. Duftstoffen wesentlich verbessert und deren dosierte Abgabe über einen langen Zeitraum erreicht wird.

Erfindungsgemäß wird diese Aufgabe mit einer Parfümkomplex-Zusammensetzung gelöst, die dadurch gekennzeichnet ist, daß sie

a) mindestens ein Parfümöl,

b) mindestens ein Silikatmaterial zur reversiblen Adsorption des Parfümöls,

c) mindestens ein Alkylammoniumsalz und

d) mindestens eine ungesättigte Fettsäure enthält.

Die erfindungsgemäße Parfümkomplex-Zusammensetzung hat insbesondere den Vorteil, daß aufgrund der verbesserten Substantivität die Dosiermenge des Parfümöls um etwa ein Drittel der bisher verwendeten Menge verringert und dennoch eine langandauernde und gleichmäßige Duftstoffabgabe erzielt werden kann.

Es ist bevorzugt, daß die vorstehend genannten Bestandteile a) bis d) in den folgenden Anteilen in der erfindungsgemäßen Parfümkomplex-Zusammensetzung unabhängig voneinander enthalten sind: 0,1 bis 3 Gew.-Teile, vorzugsweise 1 Gew.-Teil, Parfümöl; 1 bis 6 Gew.-Teile, vorzugsweise 2 Gew.-Teile, Silikatmaterial; 0,01 bis 0,06 Gew.-Teile, vorzugsweise 0,04 Gew.-Teile, Alkylammoniumsalz und 0,012 bis 0,08 Gew.-Teile, vorzugsweise 0,05 Gew.-Teile, ungesättigte Fettsäure.

Das erfindungsgemäß verwendete Silikatmaterial, vorzugsweise Metallalumosilikat, Kieselsäure oder Kieselgel, dient der reversiblen Adsorption des Parfümöls. Dies bedeutet, daß sich durch Verwendung des Silikatmaterials das Parfümöl sowohl ausgezeichnet aufziehen läßt als auch nach dem Aufziehen wieder abgegeben wird.

Es wurde nun festgestellt, daß sich die Abgabe der Parfümöle dadurch über einen möglichst langen Zeitraum dosieren läßt, daß dem Silikatmaterial mindestens ein Alkylammoniumsalz beigemischt wird, welches das Silikatmaterial mit dem daran adsorbierten Parfümöl ummantelt. Die "Silikat-Parfümteilchen" werden somit durch Alkylammoniumsalz substantiv ausgerüstet und die Reversibilität des Parfümöls wird zeitlich verzögert.

Besonders gute Effekte bezüglich des Aufziehvermögens und der dosierten Abgabe des Parfümöls werden erzielt, wenn als Silikatmaterial ein pulvriges, kristallines und dreidimensional vernetztes Metallalumosilikat mit einer Porengröße von etwa $10^{-8}$ bis $10^{-7}$ mm und vorzugsweise einer Teilchengröße von nicht mehr als 15 μm, amorphe Kieselsäure und Kieselgel mit einer Teilchengröße von nicht mehr als 30

μm, vorzugsweise nicht mehr als 15 μm, und/oder einem Schüttgewicht von 350 bis 1000 g/l, vorzugsweise 350 bis 600 g/l, verwendet werden. Ferner ist bevorzugt, daß die amorphe Kieselsäure und das Kieselgel eine spezifische Oberfläche von 100 bis 1000 $m^2/g$, vorzugsweise 600 bis 800 $m^2/g$ aufweisen. Als Metallalumosilikat wird insbesondere Natrium-Aluminiumsilikat bevorzugt, vor allem Natrium-Aluminiumsilikat mit einer Teilchengröße von nicht mehr als 15 μm und einem Schüttgewicht von 500 g/l.

Die zeitlich dosierte Abgabe des reversibel an dem Silikatmaterial adsorbierten Parfümöls läßt sich insbesondere dann über einen möglichst langen Zeitraum hinauszögern, wenn als Alkylammoniumsalz ein durch die folgende allgemeine Formel (I) dargestelltes Alkylammoniumsalz verwendet wird:

$[R_1 - NH_3]^+[X^-]$     (I)

worin $R_1$ eine $C_{12}$- bis $C_{18}$- Alkylgruppe, vorzugsweise eine $C_{16}$- bis $C_{18}$- Alkylgruppe, ist und $X^-$ ein Anion einer organischen Säure, vorzugsweise Acetat, Lactat oder Benzoat und insbesondere Acetat, darstellt.

Die in der erfindungsgemäßen Parfümkomplex-Zusammensetzung enthaltene ungesättigte Fettsäure ist vorzugsweise eine $C_{15}$- bis $C_{19}$- ungesättigte Fettsäure, die bevorzugt ausgewählt ist aus Acrylsäure, Crotonsäure, Palmitoleinsäure, Ölsäure, Erucasäure, Sorbinsäure, Linolsäure, Linolensäure, Elaeostearinsäure, Elaidinsäure, Arachidonsäure, Clupanodonsäure und Docosahexaensaure. Von diesen werden Elaidinsäure, Linolsäure, Linolensäure und vor allem Ölsäure bevorzugt der Parfümkomplex-Zusammensetzung beigemischt.

Als Parfümöle können erfindungsgemäß beispielsweise Parfümöle mit blumigen, frischen, agrumigen und kosmetischen Noten verwendet werden. Es eignen sich zum Beispiel Lavendelöl, Rosenöl, Jasminöl, Neroliöl, Zitronenöl, Orangenöl, Angelikaöl, Costusöl, Irisöl, Calmusöl, Zedernöl, Sandelöl, Zimtöl, Nelkenöl, Veilchenöl, Fliederöl, Zedernöl, Melissenöl, Rosmarinöl, Thymianöl und Maiglöckchenöl oder Gemische daraus. Weiterhin können sowohl natürliche als auch synthetische etherische Öle bzw. Gemische daraus verwendet werden.

Die erfindungsgemäße Parfümkomplex-Zusammensetzung wird dadurch hergestellt, daß man die Bestandteile a) bis d) miteinander vermischt. Dabei ist es bevorzugt, daß man zunächst das Silikatmaterial mit dem Parfümöl vermischt, das Alkylammoniumsalz und die ungesättigte Fettsäure zufügt und dann die Parfümkomplex-Zusammensetzung durch gründliches Durchmischen herstellt. Gegebenenfalls können je nach Verwendungszweck der Parfümkomplex-Zusammensetzung noch weitere Komponenten einfach beigemischt werden.

Die erfindungsgemäße Parfümkomplex-Zusammensetzung findet umfangreiche Anwendung vor allem in Körperpflegemitteln, wie Haarshampoos, Cremeschaumbädern, medizinischen Duschbädern, Zahnpasten und Handreinigungsmitteln. Weiterhin kann die erfindungsgemäße Parfümkomplex-Zusammensetzung in Haushaltsprodukten, wie Feinwaschmitteln mit Weicheffekt und Weichspülern, deren Weicheffekt sie erheblich verstärkt, Wäschestärken und abrasiven Reinigern eingesetzt werden. Darüber hinaus ist sie auch als Papierpräparationsmittel, sowohl für Sanitärpapiere als auch z.B. für technische Zwecke, geeignet. Als Präparationsmittel für Sanitärpapier wird die erfindungsgemäße Parfümkomplex-Zusammensetzung pastenförmig eingestellt und beispielsweise auf die Stirnseite von Toilettenpapierrollen aufgetragen. Ebenso kann sie jedoch auch direkt in zeitlichen Abständen auf den Toilettenbeckenrand aufgetragen werden. So behandelte Toilettenpapierrollen oder Toilettenbeckenränder ersparen beispielsweise einen Duftspender. Verbrauchte Duftspender können aber auch mit der erfindungsgemäßen Parfümkomplex-Zusammensetzung regeneriert werden. Ein Papierpräparationsmittel für technische Zwecke, enthaltend die erfindungsgemäße Parfümkomplex-Zusammensetzung, wird als Formulierung beispielsweise auf kosmetische Papiere aufgesprüht, wo es eine lang anhaltende Parfümierung des Produktes bewirkt, und es kann auch zur Parfümierung von Verpackungsmitteln verwendet werden.

Bei der Verwendung eines medizinischen Duschbads, enthaltend die erfindungsgemäße Parfümkomplex-Zusammensetzung, stellte sich überraschend heraus, daß beim Abreiben des Körpers nach dem Duschen mit dem medizinischen Duschbad eine intensive Durchblutung der Haut auftrat. Dieser Effekt ist mit keinem anderen Duschbad erreichbar.

Die Beispiele erläutern die Erfindung.

Anwendungsbeispiele

## Beispiel 1: Weichspüler

| | | |
|---|---|---|
| 8 | Gew.% | Ditalg*imidazoliniummethosulfat |
| 0,04 | " | Stearylammoniumacetat |
| 0,05 | " | Ölsäure |
| 0,3 | " | Rosenöl |
| 1,0 | " | Natriumaluminiumsilikat |
| 0,0002 | " | Sicometblau S 74180 (von BASF) |
| 0,1 | " | Hydroxybenzoesäuremethylester |
| 0,1 | " | Hydroxybenzoesäurepropylester |
| ad 100 | " | Wasser |

(* zwei $C_{17}H_{35}$-Gruppen)

## Beispiel 2: Feinwaschmittel, weichspülend

| | | |
|---|---|---|
| 5 | Gew.% | Kokosfettalkoholpolyglykolether m. 7EO (d.h. 7 $CH_2CH_2O$-Einheiten) |
| 2 | " | Ditalgimidazoliniummethosulfat |
| 0,05 | " | Ölsäure |
| 0,04 | " | Stearylammoniumacetat |
| 0,3 | " | Veilchenöl |
| 1,0 | " | Natriumaluminiumsilikat |
| 0,0002 | " | Sicometblau S 74180 |
| 0,1 | " | Hydroxybenzoesäuremethylester |
| 0,1 | " | Hydroxybenzoesäurepropylester |
| ad 100 | " | Wasser |

4

Beispiel 3: Wäschestärke

|        |       |                                     |
|--------|-------|-------------------------------------|
| 15,0   | Gew.% | Kartoffelstärke                     |
| 0,05   | "     | Ölsäure                             |
| 0,04   | "     | Stearylammoniumacetat               |
| 0,3    | "     | Lavendelöl                          |
| 3,0    | "     | Kokosfettalkoholpolyglykolether m. 7EO |
| 2,0    | "     | Polyethylenglykol, MG 600           |
| 1,0    | · "   | Natriumaluminiumsilikat             |
| 0,0002 | "     | Sicometblau S 74180                 |
| 0,1    | "     | Hydroxybenzoesäuremethylester       |
| 0,1    | "     | Hydroxybenzoesäurepropylester       |
| ad 100 | "     | Wasser                              |

Beispiel 4: Duftspüler

|        |       |                                        |
|--------|-------|----------------------------------------|
| 0,12   | Gew.% | Stearylammoniumacetat                  |
| 0,15   | "     | Ölsäure                                |
| 1,0    | "     | Fliederöl                              |
| 1,2    | "     | Xanthan-Gum                            |
| 3,0    | "     | Natriumaluminiumsilikat                |
| 0,3    | "     | Kokosfettalkoholpolyglykolether m. 7EO |
| 0,0002 | "     | Sicometblau S 74180                    |
| 0,1    | "     | Hydroxybenzoesäuremethylester          |
| 0,1    | "     | Hydroxybenzoesäurepropylester          |
| ad 100 | "     | Wasser                                 |

Beispiel 5: Shampoo

|      |       |                                        |
|------|-------|----------------------------------------|
| 3,0  | Gew.% | Kokosfettalkoholpolyglykolether m. 7EO |
| 8,0  | "     | N-(Alkylaminopropyl)-betain            |
| 2,0  | "     | Polyoxyethylensorbitanmonolaurat-Perlglanzmittel |
| 0,12 | "     | Stearylammoniumacetat                  |
| 0,15 | "     | Ölsäure                                |
| 3,0  | "     | Natriumaluminiumsilikat                |

| | | |
|---|---|---|
| 0,8 | " | Jasminöl |
| 0,0002 | " | Sicometblau S 74180 |
| 0,1 | " | Hydroxybenzoesäuremethylester |
| 0,1 | " | Hydroxybenzoesäurepropylester |
| ad 100 | " | Wasser |

**Beispiel 6:** Cremebad

| | | |
|---|---|---|
| 4,0 | Gew.% | Stearylammoniumacetat |
| 1,0 | " | Laurinsäuremonoisopropanolamid |
| 3,0 | " | Polyoxyethylensorbitanmonolaurat- Perlglanzmittel |
| 2,0 | " | Polyoxyethylensorbitammonolaurat |
| 0,15 | " | Ölsäure |
| 1,0 | " | Zedernöl |
| 3,0 | " | Natriumaluminiumsilikat |
| 0,0002 | " | Sicometblau S 74180 |
| 0,1 | " | Hydroxybenzoesäuremethylester |
| 0,1 | " | Hydroxybenzoesäurepropylester |
| ad 100 | " | Wasser |

**Beispiel 7:** Medizinisches Duschbad

| | | |
|---|---|---|
| 5,0 | Gew.% | Polyoxyethylensorbitammonolaurat |
| 3,0 | " | N-(Alkylamidopropyl)-betain |
| 0,8 | " | Stearylammoniumacetat |
| 1,0 | " | Ölsäure |
| 3,0 | " | je 1,0 Gew.-% Rosmarinöl, Melissenöl und Thymianöl |
| 3,0 | " | Isopropylmyristat |
| 20,0 | " | Natriumaluminiumsilikat |
| 0,0002 | " | Sicometblau S 74180 |
| 0,1 | " | Hydroxybenzoesäuremethylester |
| 0,1 | " | Hydroxybenzoesäurepropylester |
| ad 100 | " | Wasser |

Beispiel 8: Handreinigungsmittel

| 5,0 | Gew.% | Eiweißhydrolysat, Lampon S |
| 2,0 | " | Orangenöl |
| 0,5 | " | Kokosfettsäurediethanolamid |
| 2,0 | " | Dodecylpolyglykolether m. 4EO |
| 0,5 | " | Xanthan-Gum |
| 0,5 | " | Ölsäure |
| 0,4 | " | Stearylammoniumacetat |
| 10,0 | " | Natriumaluminiumsilikat |
| 2,0 | " | N-(Dodecylamidopropyl)-betain |
| 0,0002 | " | Sicometblau S 74180 |
| 0,1 | " | Hydroxybenzoesäuremethylester |
| 0,1 | " | Hydroxybenzoesäurepropylester |
| ad 100 | " | Wasser |

Beispiel 9: Zahnpasta

| 30,0 | Gew% | Kreide (Calciumcarbonat) |
| 10,0 | " | Glycerin |
| 0,3 | " | Stearylammoniumfluorid |
| 0,3 | " | Minzöl |
| 0,04 | " | Stearylammoniumacetat |
| 0,05 | " | Ölsäure |
| 5,0 | " | Natriumlaurylsulfat |
| 1,0 | " | Natriumaluminiumsilikat |
| 0,0002 | " | Sicometblau S 74180 |
| 0,1 | " | Hydroxybenzoesäuremethylester |
| 0,1 | " | Hydroxybenzoesäurepropylester |
| ad 100 | " | Wasser |

**Beispiel 10:** Flüssiges Scheuermittel

| | | |
|---|---|---|
| 50,0 | Gew.% | Kreide (Calciumcarbonat) |
| 0,3 | " | Xanthan-Gum |
| 2,0 | " | Kokosfettalkoholpolyglykolether m. 7EO |
| 1,0 | " | Zitronenöl |
| 0,12 | " | Stearylammoniumacetat |
| 0,15 | " | Ölsäure |
| 3,0 | " | Natriumaluminiumsilikat |
| 0,0002 | " | Sicometblau S 74180 |
| 0,1 | " | Hydroxybenzoesäuremethylester |
| 0,1 | " | Hydroxybenzoesäurepropylester |
| ad 100 | " | Wasser |

**Beispiel 11:** Papierpräparationsmittel für technische Zwecke

| | | |
|---|---|---|
| 2,0 | Gew.% | Ditalgimidazoliniummethosulfat |
| 0,08 | " | Stearylammoniumacetat |
| 0,1 | " | Ölsäure |
| 0,5 | " | Zitronenöl |
| 2,0 | " | Natriumaluminiumsilikat |
| 0,2 | " | Xanthan-Gum |
| 0,1 | " | Hydroxybenzoesäuremethylester |
| 0,1 | " | Hydroxybenzoesäurepropylester |
| ad 100 | " | Wasser |

**Beispiel 12:** Präparationsmittel für Sanitärpapier

| | | |
|---|---|---|
| 2,0 | Gew.% | Ditalgimidazoliniummethosulfat |
| 0,12 | " | Stearylammoniumacetat |
| 0,15 | " | Ölsäure |
| 1,0 | " | Zedernöl |
| 2,0 | " | Polyethylenglykol MG 600 |
| 3,0 | " | Natriumaluminiumsilikat |

```
0,8      "     Hydroxyethylcellulose
0,1      "     Hydroxybenzoesäuremethylester
0,1      "     Hydroxybenzoesäurepropylester
ad 100   "     Wasser
```

Bei allen in den Anwendungsbeispielen 1 bis 12 genannten Produkten wurde ein erhöhtes Parfümaufziehvermögen auf Haut und Haare beziehungsweise Wäsche sowie eine dosierte Abgabe der Parfümöle beobachtet. Die Dosiermengen der verschiedenen Parfümöle konnten daher um etwa ein Drittel der bisher benötigten Mengen reduziert werden. Überraschend wurde weiterhin festgestellt, daß der Weicheffekt von mit der erfindungsgemäßen Parfümkomplex-Zusammensetzung hergestellten Waschmitteln und Weichspülern ( Anwendungsbeispiele 1,2 und 4) erheblich verstärkt war.

**Patentansprüche**

1. Parfümkomplex-Zusammensetzung, **dadurch gekennzeichnet**, daß sie
   a) mindestens ein Parfümöl,
   b) mindestens ein Silikatmaterial zur reversiblen Adsorption des Parfümöls,
   c) mindestens ein Alkylammoniumsalz und
   d) mindestens eine ungesättigte Fettsäure
   enthält.

2. Parfümkomplex-Zusammensetzung nach Anspruch 1, **dadurch gekennzeichnet**, daß sie 0,1 bis 3 Gew.-Teile, vorzugsweise 1 Gew.-Teil, Parfümöl enthält.

3. Parfümkomplex-Zusammensetzung nach Anspruch 1 oder 2, **dadurch gekennzeichnet**, daß sie 1 bis 6 Gew.-Teile, vorzugsweise 2 Gew.-Teile, Silikatmaterial enthält.

4. Parfümkomplex-Zusammensetzung nach einem der Ansprüche 1 bis 3, **dadurch gekennzeichnet**, daß sie 0,01 bis 0,06 Gew.-Teile, vorzugsweise 0,04 Gew.-Teile, Alkylammoniumsalz enthält.

5. Parfümkomplex-Zusammensetzung nach einem der Ansprüche 1 bis 4, **dadurch gekennzeichnet**, daß sie 0,012 bis 0,08 Gew.-Teile, vorzugsweise 0,05 Gew.-Teile, ungesättigte Fettsäure enthält.

6. Parfümkomplex-Zusammensetzung nach einem der Ansprüche 1 bis 5, **dadurch gekennzeichnet**, daß das Silikatmaterial Metallalumosilikat, Kieselsäure oder Kieselgel ist.

7. Parfümkomplex-Zusammensetzung nach Anspruch 6, **dadurch gekennzeichnet**, daß das Metallalumosilikat ein pulvriges, kristallines und dreidimensional vernetztes Metallalumosilikat mit einer Porengröße von etwa $10^{-8}$ bis $10^{-7}$ mm ist.

8. Parfümkomplex-Zusammensetzung nach Anspruch 6, **dadurch gekennzeichnet**, daß das Metallalumosilikat eine Teilchengröße von nicht mehr als 15 $\mu$m aufweist.

9. Parfümkomplex-Zusammensetzung nach Anspruch 6, **dadurch gekennzeichnet**, daß das Metallalumosilikat Natrium-Aluminiumsilikat ist.

10. Parfümkomplex-Zusammensetzung nach Anspruch 6, **dadurch gekennzeichnet**, daß die amorphe Kieselsäure und das Kieselgel eine Teilchengröße von nicht mehr als 30 $\mu$m aufweisen.

11. Parfümkomplex-Zusammensetzung nach Anspruch 10, **dadurch gekennzeichnet**, daß die Teilchengröße nicht mehr als 15 $\mu$m beträgt.

12. Parfümkomplex-Zusammensetzung nach Anspruch 6, **dadurch gekennezeichnet**, daß die amorphe Kieselsäure und das Kieselgel ein Schüttgewicht von 350 bis 1000 g/l aufweisen.

**13.** Parfümkomplex-Zusammensetzung nach Anspruch 12, **dadurch gekennzeichnet**, daß das Schüttgewicht 350 bis 600 g/l beträgt.

**14.** Parfümkomplex-Zusammensetzung nach Anspruch 6, **dadurch gekennzeichnet**, daß die amorphe Kieselsäure und das Kieselgel eine spezifische Oberfläche von 100 bis 1000 $m^2$/g aufweisen.

**15.** Parfümkomplex-Zusammensetzung nach Anspruch 14, **dadurch gekennzeichnet**, daß die spezifische Oberfläche 600 bis 800 $m^2$/g beträgt.

**16.** Parfümkomplex-Zusammensetzung nach einem der Ansprüche 1 bis 15, **dadurch gekennzeichnet**, daß das Alkylammoniumsalz dargestellt ist durch die folgende allgemeine Formel (I):

$$[R_1 - NH_3]^+[X^-] \qquad (I)$$

worin $R_1$ eine $C_{12}$- bis $C_{18}$-Alkylgruppe und $X^-$ ein Anion einer organischen Säure ist.

**17.** Parfümkomplex-Zusammensetzung nach Anspruch 16, **dadurch gekennzeichnet**, daß $R_1$ in der allgemeinen Formel (I) eine $C_{16}$- bis $C_{18}$-Alkylgruppe ist.

**18.** Parfümkomplex-Zusammensetzung nach Anspruch 16, **dadurch gekennzeichnet**, daß $X^-$ in der allgemeinen Formel (I) Acetat, Lactat oder Benzoat ist.

**19.** Parfümkomplex-Zusammensetzung nach einem der Ansprüche 1 bis 18, **dadurch gekennzeichnet**, daß die ungesättigte Fettsäure eine $C_{15}$- bis $C_{19}$- ein- oder mehrfach ungesättigte Fettsäure ist.

**20.** Parfümkomplex-Zusammensetzung nach Anspruch 19, **dadurch gekennzeichnet**, daß die ungesättigte Fettsäure ausgewählt ist aus Acrylsäure, Crotonsäure, Palmitoleinsaure, Ölsäure, Erucasäure, Sorbinsäure, Linolsäure, Linolensäure, Elaeostearinsäure, Elaidinsäure, Arachidonsäure, Clupanodonsäure und Docosahexaensäure.

**21.** Parfümkomplex-Zusammensetzung nach einem der Ansprüche 1 bis 20, **dadurch gekennzeichnet**, daß das Parfümöl ausgewählt ist aus Lavendelöl, Rosenöl, Yasminöl, Neroliöl, Zitronenöl, Orangenöl, Angelikaöl, Costusöl, Irisöl, Calmusöl, Zedernöl, Sandelöl, Zimtöl, Nelkenöl, Veilchenöl, Fliederöl, Zedernöl, Melissenöl, Rosmarinöl, Thymianöl, Maiglöckchenöl und natürlichen oder synthetischen etherischen Ölen oder Gemischen daraus.

**22.** Verwendung der Parfümkomplex-Zusammensetzung nach mindestens einem der Ansprüche 1 bis 21 in einem Weichspüler, Feinwaschmittel mit Weicheffekt, Duftspüler, Shampoo, Cremeschaumbad, medizinischen Duschbad, Handreinigungsmittel, Papierpräparationsmittel, abrasivem Reiniger, in einer Zahnpasta oder in Wäschestärke.

| | Europäisches Patentamt | EUROPÄISCHER RECHERCHENBERICHT | Nummer der Anmeldung EP 94 10 0522 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| Y | WO-A-92 18601 (MINNESOTA MINING AND MANUFACTURING COMPANY) 29. Oktober 1992 <br> * Seite 28 - Seite 30; Beispiel 1 * <br> * Seite 33 - Seite 34; Beispiel IV * <br> --- | 1,6,16 | A61K7/46 <br> A61K7/48 |
| Y | EP-A-0 294 206 (UNILEVER PLC) 7. Dezember 1988 <br> * Seite 3 - Seite 4; Beispiel 1 * <br> * Ansprüche 1-3 * <br> --- | 1,6,16 | |
| A | EP-A-0 313 307 (THE PROCTER AND GAMBLE COMPANY) 26. April 1989 <br> * Seite 10; Beispiel 1 * <br> --- | 1-22 | |
| A | GB-A-1 306 924 (CHAS, ZIMMERMANN AND COMPANY LTD) 14. Februar 1973 <br> * das ganze Dokument * <br> --- | 1-22 | |
| A | DE-A-40 09 347 (BEIERSDORF AG) 26. September 1991 <br> * Seite 4; Beispiel 2 * <br> ----- | 1-22 | |

RECHERCHIERTE SACHGEBIETE (Int.Cl.5)

A61K

Der vorliegende Recherchenbericht wurde für alle Patentansprüche erstellt

| Recherchenort | Abschlußdatum der Recherche | Prüfer |
|---|---|---|
| DEN HAAG | 22. April 1994 | Boulois, D |